# DEMANDE DE BREVET EUROPEEN

(11) **EP 4 293 687 A1**
(43) Date de publication de la demande: **20.12.2023**
(21) Numéro de dépôt: 23179355.5
(22) Date de dépôt: 14.06.2023
(51) Int. Cl.: G21F 9/00, A61K 8/64, A61L 2/18

(54) **LINGETTE DE DÉCONTAMINATION RADIOLOGIQUE**

(30) Priorité: 17.06.2022 FR 2205932
(71) Demandeur: Ouvry SAS, 69009 Lyon (FR)
(72) Inventeur: POLLET, Thierry, 69009 C/O OUVRY SAS, 24 av. J.Masset - 69009 Lyon (FR); CHERPIN, Soizic, 69009 C/O OUVRY SAS, 24 av. J.Masset - 69009 Lyon (FR); OUVRY, Ludovic, 69009 C/O OUVRY SAS, 24 av. J.Masset - 69009 Lyon (FR); MERCIER, Julie, 69009 C/O OUVRY SAS, 24 av. J.Masset - 69009 Lyon (FR)
(74) Mandataire: Bouvier, Thibault

(57) **Abrégé**

Lingette de décontamination radiologique, pour décontaminer une surface lisse ou avec aspérités, contaminée par une contamination radiologique sous forme liquide ou solide, de poussières ou de particules,
la lingette comprenant des fibres cellulosiques naturelles ou artificielles, hydrophiles, sous forme non-tissée, et imprégnés d'une solution de décontamination radiologique dégraissante au pH neutre, exempte de phosphate, de MDTA et de EDTA, et comprenant entre 15 et 40% d'eau en masse, à un taux d'imprégnation inférieur à son taux de saturation ;
les fibres cellulosiques de la lingette permettant d'en extraire la solution de décontamination par contact sous pression ; la solution de décontamination permettant de détacher les particules contaminées, la lingette permettant de réabsorber la solution de décontamination par capillarité et absorption, et de fixer la contamination au sein de celle-ci, et de ne pas transférer la contamination ni par étalement ni par re déposition.

## Description

La présente invention concerne le domaine de la décontamination radiologique, par exemple dans le cadre de dépollution et en particulier dans le cadre de menaces NRBCe, pour tout opérateur d'intervention : défense, sécurité publique et civile (police, gendarmes), primo-intervenants (pompiers, agents hospitaliers de la santé, opérateurs industriels, personnels des sites d'infrastructures et de transports publics, etc.)

La contamination radioactive peut être définie comme la présence indésirable, à un niveau significatif, de substances radioactives à la surface ou à l'intérieur d'un milieu quelconque. L'objet contaminé devient à son tour contaminant et peut provoquer une irradiation continue et durable ; d'où l'importance et le besoin d'un moyen de décontamination de ces surfaces.

Outre les risques « classiques » de contamination dans le domaine médical ou l'industrie nucléaire par exemple, il existe aujourd'hui un risque notamment terroriste basé sur l'emploi de bombes dites « bombes sales » non conventionnelles, qui utilisent des radioisotopes largement employés pour des usages scientifiques, industriels ou médicaux, et qui sont associés à un explosif, vecteur de leur dissémination.

On trouve par exemple les éléments suivants : cobalt 60, strontium 90, césium 137, iridium 192, polonium 210, radium 226, plutonium 238, américium 241, californium 252, polonium 210 et radium 226.

Selon le spectre d'émission, on distingue plusieurs types de radioactivité selon le type de rayonnement émis :
- le rayonnement Gamma, de haute énergie, très pénétrant mais peu ionisant pour le sujet contaminé ;
- le rayonnement Beta, qui délivre une énergie plus faible sur une distance qui varie avec l'énergie (typiquement de quelques mm à quelques cm) ; et
- le rayonnement Alpha, qui délivre une énergie élevée sur une faible distance (quelques microns à quelques dizaines de microns), mais donc de façon très intense.

Au-delà d'un éventuel risque explosif, le simple contact d'un radionucléide avec une surface contamine celle-ci, que la surface soit de la peau, du textile, du matériel, etc.

La contamination, dite « externe » dans ce cas, correspond au dépôt d'un radionucléide par exemple sur les vêtements d'une personne ou directement sur la peau ou les cheveux. Elle nécessite, en amont du déshabillage, une décontamination permettant de minimiser le risque pour la santé de l'individu.

La décontamination cutanée apparait comme une étape essentielle car elle permet de diminuer la dose reçue par la peau et le passage percutané du radionucléide, de prévenir l'apparition des effets aigus et retardés radio-induits, et d'éviter la contamination croisée d'autres personnes.

L'objectif de la décontamination radiologique est donc de retirer le plus possible le contaminant de la peau ou autres surfaces, si possible le plus rapidement possible.

La décontamination radiologique peut être mise en oeuvre par lavage à l'eau et au savon, éventuellement suivie de l'application de produits décontaminant. Toutefois, en fonction du contexte environnemental de la décontamination, il n'est pas possible d'avoir accès à l'eau.

Il existe sur le marché divers dispositifs de décontamination, par exemple des lingettes de décontamination radiologique, par exemple telles que décrites à l'adresse https://www.cmecorp.com/biodex-005-301-radiacwash-dispenser-wipes-75-cnt.html ; mais aussi des gels, des crèmes, des faisceaux laser ou des détergents. Mais toutes ces solutions présentent divers inconvénients, notamment :
- brûlures de la peau pour les lingettes citées ci-dessus,
- des gels sont incompatibles avec la peau,
- des crèmes dont la gamme de température de stockage comprise entre 15°C et 25°C est très contraignante,
- l'emploi d'un faisceau laser est très limité, et impossible sur de la peau,
- les détergents nécessitent un rinçage à l'eau et sont dangereux pour la peau.

La présente invention vise à pallier ces inconvénients, et à limiter la contamination cutanée directe et l'internalisation des radionucléides dans l'organisme de l'utilisateur grâce à une lingette de décontamination.

Dans ce contexte, la présente invention concerne, selon un premier de ses objets, une lingette de décontamination radiologique, pour décontaminer une surface lisse ou avec aspérités, contaminée par un radionucléide sous forme liquide ou solide, de poussières ou de particules, la lingette comprenant des fibres cellulosiques naturelles ou artificielles, hydrophiles, sous une forme non-tissée, exempte de colle et imprégnée d'une solution de décontamination radiologique permettant de détacher les particules contaminées de la surface à décontaminer sur laquelle la lingette est appliquée, ladite solution de décontamination étant une solution dégraissante au pH neutre, exempte de phosphate, de MDTA et de EDTA, et comprenant entre 15 et 40% d'eau en masse ; la lingette étant imprégnée de ladite solution de décontamination radiologique à un taux d'imprégnation inférieur à son taux de saturation ;
les fibres cellulosiques de la lingette permettant d'en extraire la solution de décontamination par pression de la lingette sur la surface à décontaminer ;
la lingette permettant de réabsorber la solution de décontamination par capillarité et par absorption, et de fixer la contamination au sein de celle-ci, et de ne pas transférer la contamination ni par étalement ni par re déposition.

On peut prévoir que la lingette comprend en outre des fibres synthétiques, dont la forme permet une augmentation de la surface spécifique par rapport à une fibre de forme cylindrique.

En particulier, on peut prévoir que l'une au moins parmi les fibres cellulosiques et les fibres synthétiques est fonctionnalisée par greffage, configuré pour fixer les métaux lourds ionisés.

Dans un mode de réalisation, la solution de décontamination comprend du Gamma Butyrolactone.

On peut prévoir que la solution de décontamination comprend en outre au moins du tri éthylène glycol.

De préférence, la solution de décontamination est configurée pour décontaminer des radionucléides émettant dans le spectre alpha, beta ou gamma, couvrant ainsi le spectre de la radioactivité.

De préférence, les dimensions d'une lingette sont configurées de sorte que la surface de la lingette dépliée est comprise entre 500 cm² et 600 cm².

Selon un autre de ses objets, l'invention concerne une utilisation d'une lingette selon l'invention, pour la décontamination d'une surface contaminée comprenant de la peau humaine et animale.

Selon un autre de ses objets, l'invention concerne un système de décontamination radiologique, comprenant une lingette selon l'invention, et un emballage étanche souple, fin, comprenant une pluralité de couches de films polymères colaminés, limitant l'évaporation par perméation chimique, la lingette étant contenue dans l'emballage.

Enfin, selon un autre de ses objets, l'invention concerne également un kit de décontamination radiologique, comprenant un système de décontamination radiologique selon l'invention, et un ensemble d'au moins une lingette absorbante sèche.

En fonctionnement, la lingette selon l'invention agit selon deux actions principales :
- une première action, qui est une action chimique de la solution de décontamination qui favorise le décollement des particules de la surface à décontaminer, et
- une deuxième action, qui est une action mécanique de la lingette qui élimine les particules détachées de la surface par transfert.

Ainsi, la présente invention permet d'éviter d'avoir un effluent à traiter, ce qui est particulièrement avantageux dans les lieux sans eau à proximité.

La présente invention est donc une alternative à l'utilisation de l'eau et du savon.

En outre, grâce à la capacité d'absorption de la lingette, la présente invention permet une gestion des risques combinés radiologiques et chimiques.

D'autres caractéristiques et avantages de la présente invention apparaîtront plus clairement à la lecture de la description suivante donnée à titre d'exemple illustratif et non limitatif et faite en référence aux figures annexées.

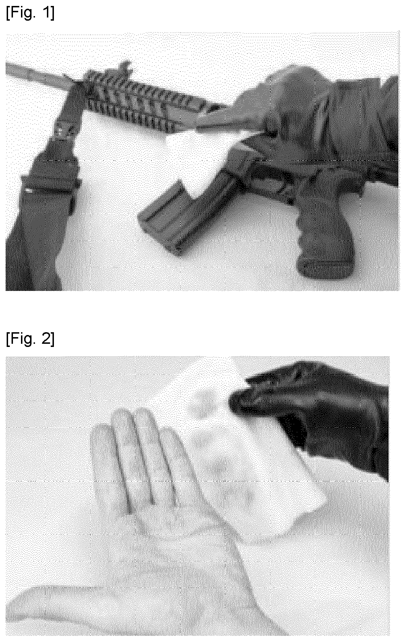

[Fig. 1] est une photographie illustrant l'utilisation d'une lingette de décontamination selon l'invention sur une arme contaminée.
[Fig. 2] est une photographie illustrant l'utilisation d'une lingette de décontamination selon l'invention sur une main contaminée.

Une lingette de décontamination radiologique selon l'invention, ci-après « lingette » par concision, est avantageusement conforme au respect du règlement REACH.

Elle comprend des fibres cellulosiques sous forme non-tissée, en l'espèce « spunlace », extrudées à l'état fondu, disposées en une couche unique. L'épaisseur de la couche peut être comprise entre 0.1 mm et 1 mm.

Les fibres peuvent être naturelles ou artificielles. Elles sont hydrophiles et exemptes de colle, ce qui limite les risques d'allergie en cas de contact avec la peau.

On peut prévoir en outre des fibres synthétiques, de préférence dont la forme permet une augmentation de la surface spécifique par rapport à une fibre de forme cylindrique. En l'espèce on peut prévoir des fibres dont la section transversale présente une forme creuse ou crénelée.

On peut prévoir des fibres synthétiques, par exemple en PA, PP, PE, PET, etc. seules ou en mélange.

On peut prévoir que l'une au moins parmi les fibres cellulosiques et les fibres synthétiques est fonctionnalisée par greffage, configuré pour fixer les métaux lourds ionisés.

Le greffage par voie chimique d'un polymère consiste à incorporer à sa surface de nouvelles fonctions pour modifier ses propriétés.

Le greffage est connu de l'homme du métier, par exemple du document
https://www.techniques-ingenieur.fr/base-documentaire/archives-th12/archivesplastiqueset-composites-tiaam/archive-3/polymerisation-a3040/greffage-etreticulationa3040niv10007.html ou encore
https://www.researchgate.net/figure/Exemple-de-fonctionnalisation-par-greffagecovalent_ fig22_281951077, ou encore des demande de brevet FR2695800, FR2708273, FR2657895.

Dans le cas de la présente invention, il s'agit d'apporter des fonctions d'échanges ioniques par l'apport de charges négatives qui permettent d'attirer puis fixer les ions métalliques chargés positivement.

Ce greffage peut être réalisé en deux étapes :
- création de sites radicalaires sur la chaine polymères des fibres de type cellulosiques ou synthétiques par activation chimique ou radiochimique ; et
- création d'une liaison covalente par réaction d'addition avec des molécules réactives (monomères insaturés avec des fonctions ioniques).

Afin d'améliorer la décontamination, les fibres sont imprégnées d'une solution de décontamination radiologique dégraissante dont le pH est neutre, c'est-à-dire compris entre 6 et 8.

La solution de décontamination radiologique est non toxique, exempte de solvant, de phosphate, de MDTA et de EDTA, ce qui la rend également biocompatible. Elle comprend entre 15 et 40% d'eau en masse, et avantageusement entre 20% et 25%.

Cette faible teneur en eau permet d'une part d'augmenter son employabilité dans une gamme de températures très large, entre -21 °C et +71 °C ; et d'autre part d'augmenter sa durée de conservation en emballage, jusqu'à 5 ans.

Par ailleurs, la lingette est imprégnée d'une solution de décontamination radiologique.

La solution de décontamination est configurée pour décontaminer des radionucléides émettant dans le spectre alpha, beta ou gamma, soit tout le spectre de la radioactivité indépendamment de la bande spectrale, et par exemple au moins l'un des radionucléides parmi : Lanthane 140, cobalt 60, strontium 90, césium 137, iridium 192, polonium 210, radium 226, plutonium 238, américium 241, californium 252, polonium 210 et radium 226.

Le taux d'imprégnation de la lingette, c'est-à-dire la quantité de masse relative de solution de décontamination radiologique par rapport à celle de la lingette est inférieur à son taux de saturation, c'est-à-dire le taux d'imprégnation maximal au-delà duquel la lingette n'absorbe plus, ce qui limite les risques de surcontamination.

De préférence, la solution de décontamination comprend du Gamma Butyrolactone. En l'espèce, la teneur en Gamma Butyrolactone est comprise entre 5% et 10%.

De préférence, la solution de décontamination comprend en outre au moins du tri éthylène glycol, qui est un additif, un surgraissant de la peau, réputé pour sa biocompatibilité, limitant les risques d'allergies. En l'espèce, la teneur en tri éthylène glycol est comprise entre 75 et 85%.

La solution de décontamination est « prête à l'emploi », c'est-à-dire qu'elle ne nécessite pas de préparation ou de mélange préalable.

Les fibres cellulosiques de la lingette permettent d'en extraire la solution de décontamination par contact sous pression de la lingette sur la surface à décontaminer. De préférence, comme illustré sur les figures, la pression sur la lingette est exercée par un utilisateur protégé avec un gant.

La solution de décontamination extraite de la lingette permet de détacher, décoller les particules contaminées de la surface à décontaminer sur laquelle la lingette est appliquée, quel que soit du reste le type de surface, biologique, métallique, à base bois ou autre, que la surface soit lisse ou avec des aspérités, que la contamination radiologique soit sous forme liquide ou solide, de poussières ou de particules. La gamme d'emploi de la lingette est donc très large.

Le support fibreux de la lingette, avec ou sans structure alvéolaire, permet de stocker la solution de décontamination. Après extraction de la solution de décontamination par pression, la capillarité et la capacité d'absorption des fibres cellulosiques de la lingette permet de réabsorber la solution de décontamination contenant alors les radionucléides, et fixer les radionucléides dans les fibres de la lingette. Grâce à cette caractéristique, la lingette permet de ne pas transférer la contamination ni par étalement ni par re-déposition.

De préférence, la lingette présente une couleur claire, en l'espèce blanche. De préférence, la solution de décontamination présente également une couleur claire, en l'espèce jaune pâle. Grâce à ces caractéristiques, les toxiques absorbés sont plus facilement visibles le cas échéant.

De préférence, les dimensions d'une lingette sont telles que la surface de la lingette dépliée est comprise entre 500 cm² et 600 cm². Avantageusement ainsi une seule lingette peut décontaminer une surface de 3 m², ce qui correspond environ à la surface d'un corps humain adulte.

En l'espèce, une lingette dépliée présente des dimensions de 33 cm x 17 cm, pour une masse inférieure à 7 g. Avantageusement, une lingette est pliée sur elle-même en 4, et présente alors des dimensions de 17 cm x 9 cm, en un format compact qui lui permet d'entrer facilement dans une poche de vêtement. La forme et la taille de la lingette permet son utilisation par tout type de personnes.

De préférence, on prévoir qu'une lingette est stockée dans un emballage. On peut prévoir qu'un emballage comprend une pluralité de lingettes, en l'espèce 10 lingettes.

L'emballage est étanche, souple et fin. Il comprend une pluralité de couches de films polymères colaminés, par exemple à base de PA, PP, PE ou PET. Grâce à cette caractéristique, l'emballage limite l'évaporation par perméation chimique et permet un stockage stable sur une longue période, même soumis à de fortes variations de températures.

On peut aussi prévoir, par exemple sous forme de kit de décontamination radiologique, un ensemble comprenant un ensemble d'au moins une lingette comprise dans un emballage selon l'invention, et un ensemble d'au moins une lingette absorbante sèche.

En opération, on peut prévoir dans un premier temps d'utiliser la lingette selon l'invention, puis dans un deuxième temps de passer la lingette absorbante sèche.

Après ouverture de l'emballage et utilisation de la lingette, l'emballage peut également servir de stockage pour la lingette contaminée.

Grâce à la présente invention, la lingette présente une formulation active polyvalente pour différents types de radionucléides, sur tout type de surface, y compris les métaux ou les alliages, et les surfaces biologiques telles que la peau, humaine ou animale.

Son support fibreux voire alvéolaires permet de stocker la solution de décontamination puis de capter et fixer les radionucléides par absorption.

Avantageusement, la solution de décontamination est respectueuse des opérateurs et de l'environnement.

## Revendications

1. Lingette de décontamination radiologique, pour décontaminer une surface lisse ou avec aspérités, susceptible d'être contaminée par une contamination radiologique sous forme liquide ou solide, de poussières ou de particules,
la lingette comprenant des fibres cellulosiques naturelles ou artificielles, hydrophiles, sous forme non-tissée, exempte de colle et imprégnés d'une solution de décontamination radiologique dégraissante au pH neutre, exempte de phosphate, de MDTA et de EDTA, et comprenant entre 15 et 40% d'eau en masse ; la lingette étant imprégnée de la solution de décontamination radiologique à un taux d'imprégnation inférieur à son taux de saturation ;
les fibres cellulosiques de la lingette permettant d'en extraire la solution de décontamination par contact sous pression de la lingette sur la surface à décontaminer ; la solution de décontamination permettant de détacher les particules contaminées de la surface à décontaminer sur laquelle la lingette est appliquée,
la lingette permettant de réabsorber la solution de décontamination par capillarité et par absorption, et de fixer la contamination au sein de celle-ci, et de ne pas transférer la contamination ni par étalement ni par re déposition.

2. Lingette selon la revendication 1, comprenant en outre des fibres synthétiques, dont la forme permet une augmentation de la surface spécifique par rapport à une fibre de forme cylindrique.

3. Lingette selon la revendication 2, dans laquelle l'une au moins parmi les fibres cellulosiques et les fibres synthétiques est fonctionnalisée par greffage, configuré pour fixer les métaux lourds ionisés.

4. Lingette selon l'une quelconque des revendications précédentes, dans laquelle la solution de décontamination comprend du Gamma Butyrolactone.

5. Lingette selon l'une quelconque des revendications précédentes, dans laquelle la solution de décontamination comprend en outre au moins du tri éthylène glycol.

6. Lingette selon l'une quelconque des revendications précédentes, dans laquelle la solution de décontamination est configurée pour décontaminer des radionucléides émettant dans le spectre alpha, beta ou gamma.

7. Lingette selon l'une quelconque des revendications précédentes, dont les dimensions sont telles que la surface de la lingette dépliée est comprise entre 500 cm² et 600 cm².

8. Utilisation d'une lingette selon l'une quelconque des revendications précédentes, pour la décontamination d'une surface contaminée.

9. Système de décontamination radiologique, comprenant une lingette selon l'une quelconque des revendications 1 à 7, et un emballage étanche souple, fin, comprenant une pluralité de couches de films polymères colaminés, limitant l'évaporation par perméation chimique, la lingette étant contenue dans l'emballage.

10. Kit de décontamination radiologique, comprenant un système de décontamination radiologique selon la revendication 9 et un ensemble d'au moins une lingette absorbante sèche.
